(19) Europäisches Patentamt
European Patent Office
Office européen des brevets

(11) Publication number: **0 584 568 A1**

(12) # EUROPEAN PATENT APPLICATION

(21) Application number: **93112075.2**

(51) Int. Cl.$^5$: **G01N 27/12**, G01N 31/00

(22) Date of filing: **28.07.93**

(30) Priority: **25.08.92 US 934937**

(43) Date of publication of application:
**02.03.94 Bulletin 94/09**

(84) Designated Contracting States:
**DE FR GB**

(71) Applicant: **EASTMAN KODAK COMPANY**
**343 State Street**
**Rochester, New York 14650-2201(US)**

(72) Inventor: **Marrese, Carl Anthony, c/o**
**EASTMAN KODAK COMPANY**
**Patent Legal Staff,**
**343 State Street**
**Rochester, New York 146501-2201(US)**
Inventor: **Chatterjee, Dilip Kumar, c/o**
**EASTMAN KODAK COMPANY**
**Patent Legal Staff,**
**343 State Street**
**Rochester, New York 146501-2201(US)**
Inventor: **Royster, Tommie Lee, Jr., c/o**
**EASTMAN KODAK CO.**
**Patent Legal Staff,**
**343 State Street**
**Rochester, New York 146501-2201(US)**
Inventor: **Paz-Pujalt, Gustavo Roberto, c/o**
**EASTMAN KODAK CO.**
**Patent Legal Staff,**
**343 State Street**
**Rochester, New York 146501-2201(US)**
Inventor: **Nicholas, Ralph Alexander, c/o**
**EASTMAN KODAK CO.**
**Patent Legal Staff,**
**343 State Street**
**Rochester, New York 146501-2201(US)**

(74) Representative: **Brandes, Jürgen, Dr. rer. nat. et al**
**Wuesthoff & Wuesthoff**
**Patent- und Rechtsanwälte**
**Schweigerstrasse 2**
**D-81541 München (DE)**

(54) **System of detecting and measuring sulfides in a sample.**

(57) A method for determining the amount of sulfide in a sample includes dissolving the sample in an acidic solution (**101**) and liberating the gas from the solution containing the dissolved sample. The gas liberated from the solution is then sensed for the presence of hydrogen sulfide therein. The sensing of the liberated gas for the presence of hydrogen sulfide may be accomplished by exposing a sensor (**19**,**300**,**405**,**421**) to the liberated gas and detecting a change in a physical property of the sensor. If hydrochloric or hydrobromic acid is used as the acidic solution, an electrochemical sensor (**300**) or a metal oxide solid state sensor (**19**) may be used to sense for the presence of hydrogen sulfide. If hydroiodic acid is used as the acidic solution, a metal oxide solid state sensor (**19**) may be used to sense the presence of hydrogen sulfide gas in the liberated gas. This technique of measuring the amount of sulfide by sensing the gas liberated from the solution using an electrochemical or metal oxide sensor provides high precision measurements of the concentration of sulfide in the sample.

EP 0 584 568 A1

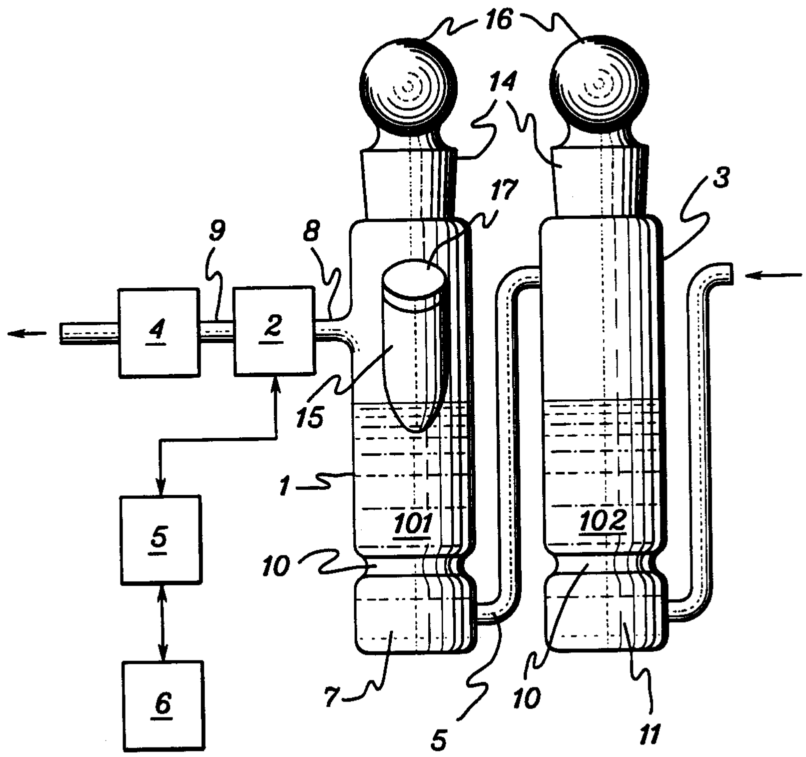


fig. 1

## Background of the Invention

The presence of sulfides in certain chemical processes may cause undesirable chemical reactions which adversely affect the desired process. For example, when silver halide is used for imaging purposes via the interaction of light with silver halide grains, sulfide, from metal sulfides, will react with silver halide to form silver sulfide. The reaction of the silver halide decreases the amount of silver halide grains available to interact with light. Therefore, it is necessary to minimize the amount of sulfide in materials used in photographic film and paper manufacturing as well as other processes.

In order to minimize the presence of sulfides, it is necessary to detect and measure their concentration. Current analytical methods for the determination of sulfide focus on solutions of uncomplexed, or unbound, sulfide ion. Common techniques, such as volumetric titrations with Cd(II) or Pb(II), determine sulfide concentrations down to the 1mM range, while direct potentiometry with an $Ag_2S$ ion selective electrode has been employed for sulfide ion concentrations from 1mM to $10^{-6}$ M, and cathodic stripping voltammetry can be used for concentrations below $10^{-6}$ M. Unfortunately, these techniques will not determine the "total" sulfide concentration, i.e., the sum of ionic and bound sulfide. In other words, it can be inferred that, if sulfide ion is detected by any of the above techniques, it is reasonable to assume that its concentration has exceeded the concentration of binding trace metal ions. Thus, to determine the total sulfide concentration one must account for the sulfide that is in the form of metal sulfides.

A conventional sample preparation technique for the liberation of sulfide from common transition metal sulfides involves reaction of the sample with mineral acid, such as hydrochloric acid (HCl) or hydroiodic acid (HI). Sulfides within the sample will react with the acid to form hydrogen sulfide ($H_2S$) which is liberated from the solution by flushing with an inert gas such as nitrogen. The liberated hydrogen sulfide is trapped by flowing the liberated gas through a buffer, such as a standard antioxidant buffer (SAOB) of pH $\approx$ 13, or a dye forming solution. The buffer solution is then usually analyzed for sulfide content by electrochemical techniques such as Cathodic Stripping Voltammetry (CSV). The dye solution is generally analyzed for sulfide content by spectrophotometry.

One problem with conventional sulfide measurement techniques is that the recovery of sulfides may vary, yielding imprecise measurement results. Typically, the precision of results is between 10% and 20%. In addition, the sensitivities of all but the CSV technique are low. The amount of sulfide capable of being analyzed by spectrophotometry is generally greater than about 50 nanograms of sulfide per gram of sample tested.

It is therefore an object of the present invention to provide a precise and accurate technique for measuring total sulfide in a sample.

It is also an object of the present invention to provide a precise and accurate technique for measuring trace amounts of sulfide in a sample.

It is also an object of the present invention to provide a precise and accurate technique for measuring sulfide bound in a sample by metal ions and for measuring sulfide trapped in a complex matrix.

## Summary of the Invention

The aforementioned objects, features and advantages may be achieved by implementation of the inventive technique for determining the amount of sulfides in a sample.

In one aspect the invention relates to a method of determining an amount of sulfide in a sample comprising:

dissolving the sample in an acidic solution;

liberating hydrogen sulfide from the solution;

exposing an electrochemical sensor or a metal oxide solid state sensor to the hydrogen sulfide liberated from the solution; and

quantifying a change in a physical property of the sensor.

Quantifying a change in a physical property of the electrochemical sensor preferably comprises measuring a voltage, or current from the sensor, preferably by means of a potentiostat. Liberating hydrogen sulfide from the solution is preferably accomplished by sparging the solution with an inert gas, preferably nitrogen. The gas may be presaturated by passing it through a gas washing bottle containing distilled water.

When the sensor is an electrochemical sensor, the sample may be dissolved in hydrobromic acid or hydrochloric acid. When the sensor is a solid state sensor, the sample may additionally be dissolved in hydroiodic acid. A preferred metal oxide solid state sensor is a tungsten oxide sensor.

In another aspect the invention relates to a method of measuring the amount of sulfide in a sample comprising:

dissolving the sample in an acidic solution;

liberating gas which evolves from the solution containing the dissolved sample;

exposing the liberated gas to a tungsten oxide sensor capable of generating a signal when exposed to hydrogen sulfide gas; and

measuring a signal from the sensor indicative of the level of hydrogen sulfide.

A preferred sensor comprises a plurality of electrodes coated by:

dissolving a compound chosen from the group consisting of:

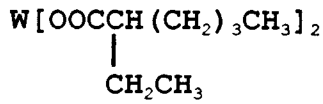

and $ClO_3W_3(OOCR)_2$ wherein R is alkyl, alkenyl or aralkyl from 2 to 19 carbons, in a solvent to form a precursor solution;

depositing the precursor solution on an electrode using a spin-coating device to form a thin film coating over the electrode; and

heating the coated electrode so that the coating decomposes to tungsten oxide.

A second preferred sensor comprises a plurality of electrodes coated by:

dissolving a compound according to the formula:

$Na[OW(OOCR)]_2$

wherein R is alkyl, alkenyl or aralkyl, from 2 to 19 carbons, in a solvent to form a precursor solution;

depositing the precursor solution on an electrode using a spin-coating device to form a thin film coating over the electrode; and

heating the coated electrode so that the coating decomposes to sodium tungsten oxide.

In a further aspect the invention relates to an apparatus for measuring sulfide in a sample comprising:

(a) a vessel containing an aqueous solution of a mineral acid;

(b) an electrochemical sensor for measuring gaseous hydrogen sulfide;

(c) a gas-carrying tube connecting the vessel, above the surface of the aqueous solution, to the electrochemical sensor; and

(d) means for sparging an inert gas through the aqueous solution and into the electrochemical sensor.

In a further aspect the invention relates to an apparatus for measuring sulfides in a sample comprising:

(a) a vessel containing an aqueous solution of a mineral acid;

(b) a metal oxide solid state sensor for measuring gaseous hydrogen sulfide;

(c) a gas-carrying tube connecting the vessel, above the surface of the aqueous solution, to the metal oxide sensor; and

(d) means for sparging an inert gas through the aqueous solution and into the metal oxide solid state sensor.

A preferred apparatus of either type additionally comprises means for presaturating the inert gas with water.

## Brief Description of the Drawings

The invention together with its objects, features and advantages will be better understood through consideration of the following detailed description in conjunction with the drawings, in which like reference numerals represent like elements, and in which:

Figure 1 depicts a schematic representation of one embodiment the system of detecting or measuring sulfides in a sample in accordance with the present invention;

Figure 2 depicts a schematic representation of a potentiostat used in the embodiment of the system of detecting or measuring sulfides in a sample in accordance with the present invention depicted in Figure 1;

Figure 3 depicts an electrochemical sensor useable to detect hydrogen sulfide and useable in the embodiment of the system of detecting or measuring sulfides in a sample in accordance with the present invention depicted in Figure 1;

Figure 4 depicts a schematic representation of a second embodiment of a system of detecting and measuring sulfides in a sample in the presence of iodide in accordance with the present invention;

Figure 5 depicts a tungsten oxide solid state sensor useable in the embodiment of the system of detecting or measuring sulfides in a sample depicted in Figure 4;
Figure 6 depicts a schematic representation of the solid state sensor electronics useable in the system of detecting or measuring sulfides in a sample incorporating a sensor such as the tungsten oxide solid state sensor depicted in Figure 4;
Figure 7 depicts a schematic representation of alternative sensor electronics useable in the system of detecting or measuring sulfides in a sample incorporating a sensor such as the tungsten oxide solid state sensor depicted in Figure 4;
Figure 8 depicts a graphical representation of various current time curves indicative of the concentration of sulfide in different samples; and
Figure 9 depicts a graphical representation of the area under the current time curves in Figure 8 as a function of sulfide concentration level.

## Detailed Description

Referring to Figures 1 and 4, the system of determining the amount of sulfide in a sample includes an evolution chamber **1**, a sensor manifold **2**, a flow meter **4** and gas washing bottle **3**. The apparatus is oriented such that an inert gas passes through the gas washing bottle where it is presaturated with water prior to entering the evolution chamber **1**. The evolution chamber **1** contains an acidic solution with a dissolved sample of a composition to be tested for the presence of sulfides. Gas is liberated from the evolution chamber **1** and passes through the sensor manifold **2** where it is sensed by a sensor for the presence of hydrogen sulfide gas.

Figure 1 depicts a schematic representation of one embodiment of the present invention; Figure 4 depicts an alternative embodiment. The embodiment depicted in Figure 1 may be used to detect the presence of sulfides in a sample not containing iodide therein, or when an acidic solution used to dissolve the sample does not contain iodide. In these situations, a conventional electrochemical hydrogen sulfide sensor may be used and mounted within the sensor manifold **2**, depicted in Figure 1. In situations where the sample and/or acidic solution contains iodide (such as hydroiodic acid) then the embodiment of the invention depicted in the apparatus shown in Figure 4 should be used to detect and/or measure the level of hydrogen sulfide in the sample. In these situations, a metal oxide solid state sensor such as a tungsten oxide solid state sensor is mounted in the sensor manifold **2**, depicted in Figure 4.

Referring again to Figure 1, an evolution chamber **1** contains a non-iodic acidic solution **101** therein. At the inlet **7** of the evolution chamber, a conduit **5** connects to a gas washing bottle **3**. At the outlet of the evolution chamber **1**, a conduit **8** connects the sensor manifold **2** with the evolution chamber. The sensor manifold **2** houses a hydrogen sulfide sensor, not shown in Figure 1 but shown in detail in Figure 3. Referring again to Figure 1, a flow meter **4** is connected to the outlet conduit **9** of the sensor manifold to measure the flow of gas through the system. The entire system provides a network for gaseous flow. An inert gas enters the inlet **11** of a gas washing bottle where it is presaturated with water as is well known in the art. The presaturated inert gas then flows through conduit **5** to the evolution chamber inlet **7** where it sparges the acidic solution containing a dissolved sample of the composition being tested for sulfide content.

The evolution chamber **1** contains an inlet orifice **14** wherein the acidic solution may be added. It is normally closed by stopper **16**. The evolution chamber **1** also contains an optional second inlet orifice **15** also normally sealed by septum **17**. The inert gas which is sparged through the acidic solution **101** will contain hydrogen sulfide gas as a result of the reaction between the acidic solution and the sulfide present in the sample. The liberated gas will enter the sensor manifold **2**, by passing through the conduit **8**. A three-way stopcock (not shown) may be optionally interposed in conduit **8** to direct the flow of gas aside or to the sensor manifold **2**, where the hydrogen sulfide electrochemical sensor will respond to the hydrogen sulfide gas. The electro-chemical reaction affects the voltage bias of the sensor. A change in voltage, or current, across the sensor is therefore indicative of the presence and/or level of hydrogen sulfide gas contained in the liberated gas. After the gas flows through the sensor, it will exit the sensor manifold **8** via tube **9** and enter the flow meter **4** which measures the flow rate of the gas. The voltage or current across the sensor is measured by a potentiostat **5** which is connected to a graphical display **6**. The display is capable of indicating, by graphical representation, the voltage or current across the sensor as a function of time.

A suitable evolution chamber is of the configuration shown in figures 1 and 4, having a fritted glass disc **10** between the inlet area **7** and the body of the vessel **1**. In addition the vessel has an optional second inlet orifice **15**, which may be capped with a stopper **16** (not shown) or a septum cap **17** for the sealed introduction of samples.

The flow meter **4** may comprise any commercially available flow meter, such as those manufactured by Cole-Parmer, which are well known in the art. The sensor manifold **2** is of the type manufactured by City Technology, Ltd (London, UK) and is described in U.S. Patents 4,587,003, 4,633,704 and 4,406,770 with modifications as discussed below.

The graphical display **6** may be any means which will measure voltage or current as a function of time including but not limited to, a two pen strip chart recorder. Other equivalent means such as an oscilloscope or digital computer which are well known in the art may be used. The gas washing bottle **3** may comprise any conventional gas washing systems which presaturate gas with water as are well known in the art. In the Figs. 1 and 4 it is shown as being constructed in similar fashion to the evolution chamber, but lacking orifice **15**. For use in the present invention, the water should be prefiltered for purity using a conventional filtering means such as a Milli-Q filtration apparatus which purifies water by an anion exchange technique with activated charcoal filtering.

The potentiostat **5** used to measure the sensor is shown in detail in Figure 2. A hydrogen sulfide sensor contact **201** is connected to the sensing (working) electrode of the hydrogen sulfide sensor while contact **203** is connected to the sensor's reference electrode. The difference in potential between contact **201** and contact **203** should be 0.0 V. Contact **205** is connected to the counter electrode of the sensor. The potentiostat employs circuitry similar to that designed by City Technology Ltd of London, England which includes integrated circuit (IC) chips **207**, **209** and **211**. In this embodiment IC chip **207** is an OP-14EZ, chip **209** is an OP-07EZ and chip **211** is an OP17EZ manufactured by Precision Monolithics, Inc. (Santa Clara, CA). Each of the resistors depicted in Figure 2 are $\frac{1}{4}$ watt ± 1% unless otherwise noted in Figure 2. The capacitance of capacitor **213** may vary depending upon the cut-off frequencies. The capacitance for each cut-off frequency in the particular embodiment described above is as follows:

| | |
|---|---|
| 0.1μF at 28HZ | 3.3 μF at 0.86HZ |
| 1 μF at 2.8HZ | 4.7 μF 0.6HZ |
| 2.2 μF at 1.3HZ | 10 μF at 0.28HZ |

Referring now to Figure 3, the components of the modified electrochemical hydrogen sulfide sensor **300** are disclosed. The sensor is of the type manufactured by City Technology Ltd. (London, UK) with modifications. The sensor cell cover was replaced with a modified inlet cover **21** to allow effluent gas from the evolution chamber to enter and exit the sensor through a standard 14/20 taper **22**. The effluent gas stream is saturated with water and HCl vapor. Therefore, to prevent condensation on the sensor working electrode **329**, a Zytex® 0.254 mm thick porous Teflon disc **23** was inserted between the modified cell inlet cover **21** and the electrode compression device **24**. The remaining parts of the cell were arranged as received from the manufacturer: O-ring **341**, cell cavity for $H_2SO_4$ electrolyte **328** and Pyrex filter "wicks" **317**.

The use of the system as depicted in Figure 1 is initiated by adding an acidic solution such as hydrochloric or hydrobromic acid to the evolution cell. An inert gas, preferably nitrogen, is flowed into the system via gas washing bottle inlet **11**. The nitrogen is presaturated with purified water **102** in the gas washing bottle **3** before it enters the evolution chamber **1** at inlet **7** where it is sparged in the acidic solution. During sparging, liberated gas exits the evolution chamber **1** via the outlet conduit **8** and passes through the sensor manifold **2** and flow meter **4**. The flow rate of nitrogen is controlled by an inlet valve (not shown) until a liberated gas flow rate of approximately 50 mL/min ± 5 mL/min is maintained as indicated by the flow meter **4**. Once this flow rate has been obtained and the output voltage or current of the sensor, as measured by the potentiostat **5** and displayed via the graphical display **6**, has established a steady state base line, the sample of the composition to be tested may be injected into the evolution chamber through septum **17**. The sample should be added to the acidic solution quickly while the voltage and/or current measured by the potentiostat and displayed by the graphical display **6** is recorded. The recorded voltage or current is directly proportional to the amount of hydrogen sulfide present. The proportional relationships can be experimentally predetermined using samples having known sulfide concentration levels or some other suitable technique.

Typically, a six molar hydrochloric solution about 28 mL or so in volume may be used in the evolution cell. The sample may be dissolved in purified water prior to insertion into the acidic solution. For example, 2 grams of a sample composition dissolved in 10 mL of purified water can be added to the 28 mL of 6M hydrochloric acid.

As the sample is added to the acidic solution of either hydrochloric or hydrobromic acid, the sulfides react with the acid to form hydrogen sulfide gas. The hydrogen sulfide gas is liberated from the acidic

solution by the inert gas (typically as prepurified 99.99999% nitrogen). The liberated gas including the hydrogen sulfide gas flows into the sensor manifold 2 and into the gas inlet port **309**, shown in Figure 3, of the hydrogen sulfide sensor. The gas enters the gas inlet port **309** and encounters Teflon film **23**. The gas diffuses through the film and contacts the platinum/carbon working electrode **329**. At this electrode, water vapor within the gas and hydrogen sulfide react according to the following equation:

$$4H_2O + H_2S \rightarrow H_2SO_4 + 8H^+ + 8e^- \quad (1)$$

At the counter electrode **315** of the sensor, oxygen reacts with the products of the above equation to form water according to the equation:

$$O_2 + 4e^- + 4H^+ \rightarrow 2H_2O \quad (2)$$

The overall reaction, as a combination of the above equations, is:

$$H_2S + 2O_2 \rightarrow H_2SO_4 \quad (3)$$

Test Results

| Conditions and Parameters | |
|---|---|
| Acidic solution: | 28 mL of 6M HCl. |
| Inert gas: | $N_2$ @ 50 mL/min ± 10%. |
| Sample: | 2 g ± .1 g NaCl dissolved in 10 mL purified $H_2O$. |
| Known sulfide content: | 60 ng $S^=$/g NaCl. |

Using the above tabulated parameters, a test run of the system was conducted, a portion of a sodium chloride sample containing 60 nanograms of sulfide per gram of sodium chloride was dissolved in 10 mL of purified water and added to the evolution cell while recording the current time curve on the graphical display. A first peak of the current time curve was recorded on the graphical display. Additional sulfide was added to the nondissolved portion of the sample to increase the total concentration of sulfide in the nondissolved sample. By adding additional sulfide using techniques well known in the art, this nondissolved sample contained 120 nanograms of sulfide per gram of NaCl. The higher concentration sample (i.e., the spike) containing the added sulfide was added to the evolution cell after the initial time current curve had reached its original base line. The graphical display then again recorded a second peak of the current time curve. The concentration of sulfide in the first added sample was calculated by the following equation:

$$ng(S^=)/g\ NaCl = \frac{(ng(S^=\ spike)/g\ NaCl)\ x\ (Area\ i_{p1})}{(Area\ i_{p2} - Area\ i_{p1})} \quad (4)$$

{tc \f E "(4) "}

Where (ng ($s^=$ spike)/g NaCl) is the concentration of the additional added sulfide concentration, $P_1$ represents the first peak or time curve and $P_2$ represents the second peak or time curve.

In quantifying the amount of sulfide in the sample, either a direct comparison method or standard addition method, each well known in the art, may be employed. The standard addition appears more accurate in the system of the invention. The standard addition method of quantification first requires that the concentration level of sulfide be linear with the peak area. Figure 8 depicts recorded current time curves for various known concentration levels, and Figure 9 depicts a plot of the peak area of the current time curves in Figure 8 as a function of concentration level. The results indicate that sulfide concentration is linear with the area under the current-time peak curve. The results from twelve replicate analyses (performed over a four-day period) by the gas-phase system employing standard addition of $Na_2S$ to an NaCl solution yielded a mean sulfide concentration of 63 ng/g with a standard deviation of 5.7 ng/g. The precision, expressed as the relative standard deviation, was 9.1%.

For practical purposes, the detection limit may be defined as the lowest concentration of sulfide yielding a peak whose amplitude is twice the amplitude of the background. With this definition we have determined that the detection limit for NaCl spiked with $Na_2S$ by the gas-phase sensor system was 10 ng/g. Typical calibration plots of peak area vs sulfide concentration have not included concentrations below 20 ng/g. These plots yield straight lines with correlation coefficients typically around 0.997 and intercepts very close to zero.

Table 1 indicates three experimental runs for testing known samples containing 60 milligrams sulfide per gram NaCl by the standard addition method.

Table 1

| Determination of $S^{=}$ in NaCl | |
|---|---|
| Sample | ng $S^{=}$/g NaCl (Standard) |
| 1 | 52 |
| 2 | 60 |
| 3 | 63 |
| Average | 58 |

Sulfide may also be detected and measured in the presence of other common salts. Solutions of NaBr, KBr, KCl, and $(NH_4)_2SO_4$ containing $Na_2S$ are easily analyzed and yield detection limits of 15 - 20ng/g. (Solutions of $(NH_4)_2SO_4$ do not exhibit the recovery problems that the alkali salts do, and therefore, can be analyzed directly by CSV). Lead nitrate forms a precipitate, presumably $PbCl_2$, and yields poorly defined peaks and, ultimately, inaccurate results. Finally, waste water samples have been analyzed with the gas-phase sensor system yielding a detection limit of 1 ng/mL.

A significant feature of the apparatus of the invention, and a major factor in its precision and accuracy, is the location of the $H_2S$ gas sensor in close operational proximity to the site at which the $H_2S$ is generated. The apparatus of the art, using $H_2S$ capture in solution, does not exhibit this particularly advantageous configuration.

If the acidic solution, used in the evolution chamber to react with the sample containing sulfides, contains iodides (i.e., hydroiodic acid), or if the test sample contains iodide, then the system depicted in Figure 4 must be used to measure sulfide levels.

Figure 4 depicts a system incorporating a tungsten oxide solid state sensor used to sense levels of hydrogen sulfide within the gas liberated in the evolution chamber **1**. This apparatus includes the evolution chamber **1** with the gas washing bottle **3**, the inert gas inlet **15**, the flow meter **4**, sensor manifold **2** and a power supply **413** along with other features similar to those depicted in Figure 1 oriented in a gas flow network. The features of the embodiment depicted in Figure 4 are identical to those of the embodiment adjusted in Figure 1, with the exception of the modifications described infra. Although the system is used when the acidic solution contains iodide therein, such as hydroiodic acid, or when the test sample contains traces of iodide, the operation of the system is similar to the operation of the system depicted in Figure 1 and discussed supra.

Solid state sensors using tungsten oxide films are known in the art. Examples may be found in U.S. patents 4,197,089 (Willis) and 4,822,465 (Jones), and British published application 2,137,356 (Bird) which are incorporated herein by reference. A method for forming a tungsten oxide film on an electrode is disclosed in U.S. patent 5,034,246 (Mance) which is also incorporated herein by reference.

A solid state sensor having tungsten oxide coated electrodes prepared using a specific precursor compound preferred for use in the present invention, is described in the continuation-in-part of co-pending U.S. patent application 07/677,729 entitled "Hydrogen Sulfide Gas Sensor and Precursor Compound for Manufacture of Same" by Royster et al.

In brief, the preferred precursors are compounds of formulas I, II and III

$Na_2[OW(OOCR)_2]_3$ **I**

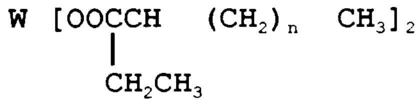

**II**

$ClO_3W_3(OOCR)_2$ **III**

wherein R is as defined earlier.

R can be any hydrocarbon chain, so long as the overall solubility and rheological properties of the tungsten carboxylate in aliphatic or aromatic hydrocarbon solvents are not significantly changed. The preferred subgenera in which R is $C_6$ to $C_{10}$ optimize the balance among solubility, rheology and reactivity of the starting acid for forming the tungsten carboxylates. The novel mixed valence tungsten (III) and (IV) carboxylates take the form of blue glassy solids and are sensitive to air and moisture. The tungsten (II) carboxylates of Formula II are dark green oils or glasses and are also moisture sensitive.

The solubility of the compounds in aliphatic and aromatic hydrocarbons makes them useful in solution casting techniques, such as spin-casting, dip-casting and spray-casting.

The tungsten carboxylates may be synthesized according to the following reactions:

1. $$2\ OWCl_4 + 8\ NaOOCR + RCOOH$$
$$\downarrow$$
$$\underset{\textbf{I}}{Na[OW(OOCR)_2]2_3} + 5\ R^1C{=}CR^2\uparrow + 5\ CO_2\uparrow + 4H_2\uparrow + 7\ NaCl + 1HCl\uparrow$$

2. $$W(CO)_6 + 2RCOOH \rightarrow \underset{\textbf{II}}{W(RCOO)_2} + H_2\uparrow + 6\ CO\uparrow$$

3. $$3\ OWCl_4 + 11\ RCOOH$$
$$\downarrow$$
$$\underset{\textbf{III}}{Cl\ O_3W_3(OOCR)]_2} + 9\ R^1{-}C{=}C{-}R^2\uparrow + 9\ CO_2\uparrow + 9/2\ H_2\uparrow + 11\ HCl\uparrow$$

In equations 1 and 3, $R^1$ and $R^2$ represent the appropriate alkyl, alkenyl or aralkyl residues that would arise from the corresponding R group.

The method of preparing the compounds of Formula I comprises the steps of reacting an alkali metal with an excess of an organic acid to form a carboxylate salt solution; reacting the carboxylate salt solution with a solution containing tungsten (VI) oxychloride in an aromatic solvent in an inert atmosphere to form a reaction mixture; refluxing the reaction mixture to form a sodium tungsten (III & IV) carboxylate, and extracting the sodium tungsten (III & IV) carboxylate from the refluxed mixture. Suitable aromatic solvents include toluene and benzene. Suitable alkali metals include sodium, potassium and lithium. Particularly suitable organic acids include 2-ethylhexanoic acid and 4-phenylbutyric acid or 3-phenylbutyric acid.

Compounds of generic Formula II are prepared in an inert atmosphere by heating tungsten hexacarbonyl with a large excess of the appropriate carboxylic acid at reflux, for acids with boiling points below 200° C, or at 200° C, for those boiling higher. The heating is maintained until all the tungsten hexacarbonyl is consumed. The solution is filtered and the excess acid is distilled off under reduced pressure.

Compounds of generic Formula III are prepared by heating tungsten oxychloride with a large excess of the appropriate carboxylic acid in an inert atmosphere at about 160°. The reaction is filtered and the excess acid is distilled off under reduced pressure. Examples of sample preparations of the compounds are set forth below.

EXAMPLE 1

Formula I, R = 1-ethylpentyl Working in a conventional dry box, 3.43 g (10.0 mmol) of tungsten (VI) oxychloride was placed in a 200 ml Schlenk flask. Toluene (65 mL) was syringed onto the sample. Freshly cut sodium (0.949 g, 41.3 mmol) was placed into a 250 mL 2-neck flask. The 2-neck flask was connected to the Schlenk flask by a bent elbow. The second neck of the 2-neck flask was stoppered using a rubber septum. Outside of the dry box, 45 mL of 2-ethylhexanoic acid was syringed onto the sodium and the mixture was heated below the boiling temperature of 113° C until the sodium had completely reacted. The adapter to the Schlenk flask was purged with nitrogen gas before opening the system to a connected bubbler. The 2-ethylhexanoic acid-salt solution was added to the tungsten (IV) oxychloride solution while stirring at room temperature. Under a purge of nitrogen gas, a condenser was connected to the Schlenk flask. The reaction mixture was refluxed using an oil bath heated at 125° C. After 16 hours, the solution was cooled to room temperature under a purge of nitrogen. The toluene was removed by vacuum distillation. To remove the excess 2-ethylhexanoic acid, a dynamic vacuum was used while heating at 110° C with an oil bath. The glassy blue product was extracted from the sodium chloride in the refluxed mixture with pentane.

EXAMPLE 2

Formula II R = 1-ethylpentyl Working outside of the dry box, 4.04 g (11.4 mmol) of $W(CO)_4$ was weighed and placed into a 240 mL Schlenk flask fabricated for refluxing reaction mixtures. Using a syringe, 80 mL (0.5 mol) of 2-ethylhexanoic acid (EHA) was added to the flask. After connecting a condenser that was attached to a nitrogen line, the reaction flask was heated using an oil bath. The temperature of the oil bath reached 195° - 200° C. Maintaining the temperature the solution was heated until $w(CO)_6$ no longer sublimed up on the walls of the flask (4 days). A dark green solution was observed. Periodically the $W(CO)_6$ was washed down from the walls by agitating the solution. Before the heat was removed, the valve on the Schlenk flask was closed to prevent air from going into the flask. Working in a dry box, the solution was filtered through a 0.45 micron cellulose acetate filter. The filtered solution was transferred to a 200 mL round bottom single- neck flask which was connected to a 250 mL Schlenk flask using a bent elbow. The excess EHA was removed by heating the dark green solution with a 130° C oil bath. A dark green material with a thick oil consistency was obtained. The infrared spectrum of the material was consistent with product of Formula II where R is 1-ethylpentyl (carbonyl at 1680 $cm^{-1}$).

EXAMPLE 3

Formula III, R = 1-ethylpentyl Working in the dry box 4.13 g (12.1 mmol) of OW $Cl_4$ was weighed into a Schlenk flask that was fabricated for refluxing reaction mixtures. Then, 60 mL (0.375 mol) of 2-ethylhexanoic acid was added to the reaction flask. Working outside of the dry box, the nitrogen purged condenser was attached to the reaction (the valve was still closed at this point). After heating the reaction flask in a 100° C bath for five minutes, the valve was opened. The condenser was connected to a bubbler and the oil bath was heated to 160° C. The reaction mixture turned deep blue/purple. It was maintained at 160° C for 24 hours. Before the heat was removed, the valve on the reaction flask was closed to prevent exposure to air. The reaction flask was taken into the dry box and the solution was filtered through a 0.45$\mu$ cellulose nitrate filter. Using pentane, the remaining material was rinsed out of the flask. A dark blue solid was collected on the filter medium. The filtered solution was transferred to a 250 mL one neck flask. The pentane was removed in vacuo and the excess acid was distilled off at 120° C under vacuum.

The material was placed in a 250 mL one neck flask. The flask was connected to a fine porosity frit and a 250 mL Schlenk flask. About 100 mL of diethyl ether was distilled onto the product in vacuo. The ether-soluble portion of the material was extracted into the 250 mL Schlenk flask. The dark blue solid product (Formula III R = ethylpentyl) was isolated by removal of the ether in vacuo.

The tungsten carboxylates provide an improved film coating of tungsten oxide on the electrodes used in hydrogen sulfide gas sensors. The film of the tungsten carboxylate precursor is applied or deposited on the electrodes, preferably arranged in an interdigitated configuration, by a known solution casting technique. This is possible because the carboxylates are soluble in the solvents used in solution casting techniques and have the necessary rheology and surface wetting properties. The resulting thin films from II and III decompose to tungsten oxide when heated to above approximately 350° C by conventional curing methods; the films from I decompose to sodium tungsten oxide.

A general procedure for coating a substrate is provided by the following example:

Working in the dry box, 50 $\mu$L of a toluene solution containing 17 mg of the precursor compound was

syringed onto a quartz glass plate. The thin film was laid down using a photoresist spinner at 2000 rpm for 20 seconds. Outside of the dry box, the quartz glass was placed on a hot plate. After 30 minutes, the glass was removed and a transparent thin film was observed.

When the temperature of the hot plate was 520 ° C, the product from the compound of example 2 was hexagonal phase $WO_3$ as shown by x-ray diffraction.

Use of a metal oxide solid state sensor such as that taught by Royster et al within the system depicted in Figure 4 requires the use of solid state sensor electronics **19** to control the power supply **13** to the solid state sensor and to process the signals obtained from the sensor so they may be converted into graphical data. Figure 5 depicts a solid state sensor having tungsten oxide ($WO_3$) coated electrodes including the connections of the heater power supply and MOS power supply. The sensor contains an alumina chip support **431** having a heater **433** with an insulated coating, an interdigitated $WO_3$ film coating **435** and contacts **405**, **407**, **409** and **411**.

Referring to Figure 5, the heater power supply **401** is connected to palladium contacts **405** and **407** of the sensor. The MOS power supply **403** is connected to tungsten oxide contacts **409** and **411**. As shown in Figure 6, a biasing power supply **413** is operatively connected to the tungsten oxide contacts **409** and **411**. A resistor **415** is connected in series with the biasing power supply and a tungsten oxide contact **411**. An operational amplifier **417** and a resistor **419** are connected to the graphic display **6**. The voltage across the tungsten oxide contacts **409** and **411** is represented by $E_1$. $E_2$ represents the voltage across the bias power supply **413** and $E_o$ represents the output voltage to the graphical display. $E_o$ is obtained by the equation:

$$E_o = -(E_1 (RF/R1) + E_2 (RF/R2)) \qquad (5)$$

The current supplied by the heater power supply to the palladium contacts should be approximately 10 microamps. The current supplied by the MOS power supply to the tungsten oxide contacts should also be approximately 10 microamps. The power supply **413** may comprise a conventional power supply manufactured by Hewlett Packard, Model #6634A. The operational amplifier used in this system may be Computer Products - PM565 or any other equivalent. The resistances R1, R2 and RF should be matched to the sensor's resistance when the sensor is at rest, i.e., in the absence of $H_2S$. Using this technique, the voltage difference $E_o$ and/or current may be recorded by the XY recorder as a function of time and the amount of sulfide sensed may be represented by the area under the curve. Similar to the techniques discussed supra, the standard addition method and/or comparison method may be used to measure the level of hydrogen sulfide.

As an alternative to the circuitry useable in the solid state sensor electronics **19** as depicted in Figure 6, the circuitry depicted in Figure 7 may also be used. In this circuitry configuration, a power supply inputs a voltage $E_{in}$ to a first tungsten oxide sensor **405** which is operatively connected to an operational amplifier **417** and second tungsten oxide solid state sensor **421**. The voltage on the output of the second tungsten oxide solid state sensor **421** and operational amplifier **417** and ground is represented by $E_{out}$. The first tungsten oxide solid state sensor **405** is exposed to the liberated gas in the sensor manifold **2** such that the resistance across the sensor changes. The second tungsten oxide solid state sensor **421** is not exposed to the liberated gas such that the resistance across this sensor remains constant. The output voltage, $E_{out}$, may be represented by the equation:

$$E_{out} = -E_{in} (R_f/R_V) \qquad (6)$$

The voltage signal $E_{out}$ can be connected to a graphical display **6** which records the voltage and/or current as a function of time in order to measure the amount of sulfide detected as previously discussed.

Although, this embodiment has been described as having a solid state sensor with tungsten oxide coated electrodes, other metal oxide solid state sensors may be incorporated into this embodiment of the invention.

Although the invention has been described in conjunction with the embodiments depicted herein, it will be apparent to one skilled in the art that the invention is not so limited. Various modifications, changes, substitutions and variations may be made to the invention without departing in any way from the spirit of the invention as defined by the following claims.

## Claims

**1.** A method of determining an amount of sulfide in a sample comprising:
dissolving said sample in an acidic solution (**101**);

liberating hydrogen sulfide from said solution;
exposing an electrochemical sensor (**300**) to said hydrogen sulfide liberated from said solution; and quantifying a change in a physical property of said sensor (**300**).

**2.** A method of determining an amount of sulfide in a sample comprising:
dissolving said sample in an acidic solution (**101**);
liberating hydrogen sulfide from said solution;
exposing a metal oxide solid state sensor (**19**) to said hydrogen sulfide liberated from said solution;
quantifying a change in a physical property of said sensor (**19**).

**3.** A method of measuring the amount of sulfide in a sample comprising:
dissolving the sample in an acidic solution (**101**);
liberating gas which evolves from the solution containing the dissolved sample;
exposing the liberated gas to a tungsten oxide sensor (**405**,**421**) capable of generating a signal when exposed to hydrogen sulfide gas; and
measuring a signal from said sensor (**405**,**421**) indicative of the level of hydrogen sulfide.

**4.** A method of measuring the amount of sulfide in a sample according to claim 14 wherein said sensor (**405**,**421**) comprises a plurality of electrodes (**329**) coated by:
dissolving a compound chosen from the group consisting of:

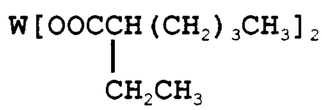

and $ClO_3W_5(OOCR)_2$ wherein R is alkyl, alkenyl or aralkyl from 2 to 19 carbons, in a solvent to form a precursor solution;
depositing the precursor solution on an electrode (**329**) using a spin-coating device to form a thin film coating over the electrode; and
heating the coated electrode so that the coating decomposes to tungsten oxide.

**5.** A method of measuring the amount of sulfide in a sample according to claim 14 wherein the electrodes (**329**) are coated by:
dissolving a compound according to the formula:

$Na[OW(OOCR)]_2$

wherein R is alkyl, alkenyl or aralkyl from 2 to 19 carbons, in a solvent to form a precursor solution;
depositing the precursor solution on an electrode (**329**) using a spin-coating device to form a thin film coating over the electrode; and
heating the coated electrode so that the coating decomposes to sodium tungsten oxide.

**6.** An apparatus for measuring sulfide in a sample comprising:
(a) a vessel **1** containing an aqueous solution of a mineral acid (**101**);
(b) an electrochemical sensor (**300**) for measuring gaseous hydrogen sulfide;
(c) a gas-carrying tube (**8**) connecting said vessel above the surface of said aqueous solution to said electrochemical sensor (**300**); and
(d) means for sparging an inert gas through said aqueous solution in said vessel 1 and into said electrochemical sensor (**300**).

**7.** An apparatus for measuring sulfide in a sample comprising:
(a) a vessel **1** containing an aqueous solution of a mineral acid (**101**);
(b) a metal oxide solid state sensor (**19**) for measuring gaseous hydrogen sulfide;
(c) a gas-carrying tube (**8**) connecting said vessel **1** above the surface of said aqueous solution to said metal oxide sensor (**19**); and

(d) means for sparging an inert gas through said aqueous solution in said vessel and into said metal oxide solid state sensor.

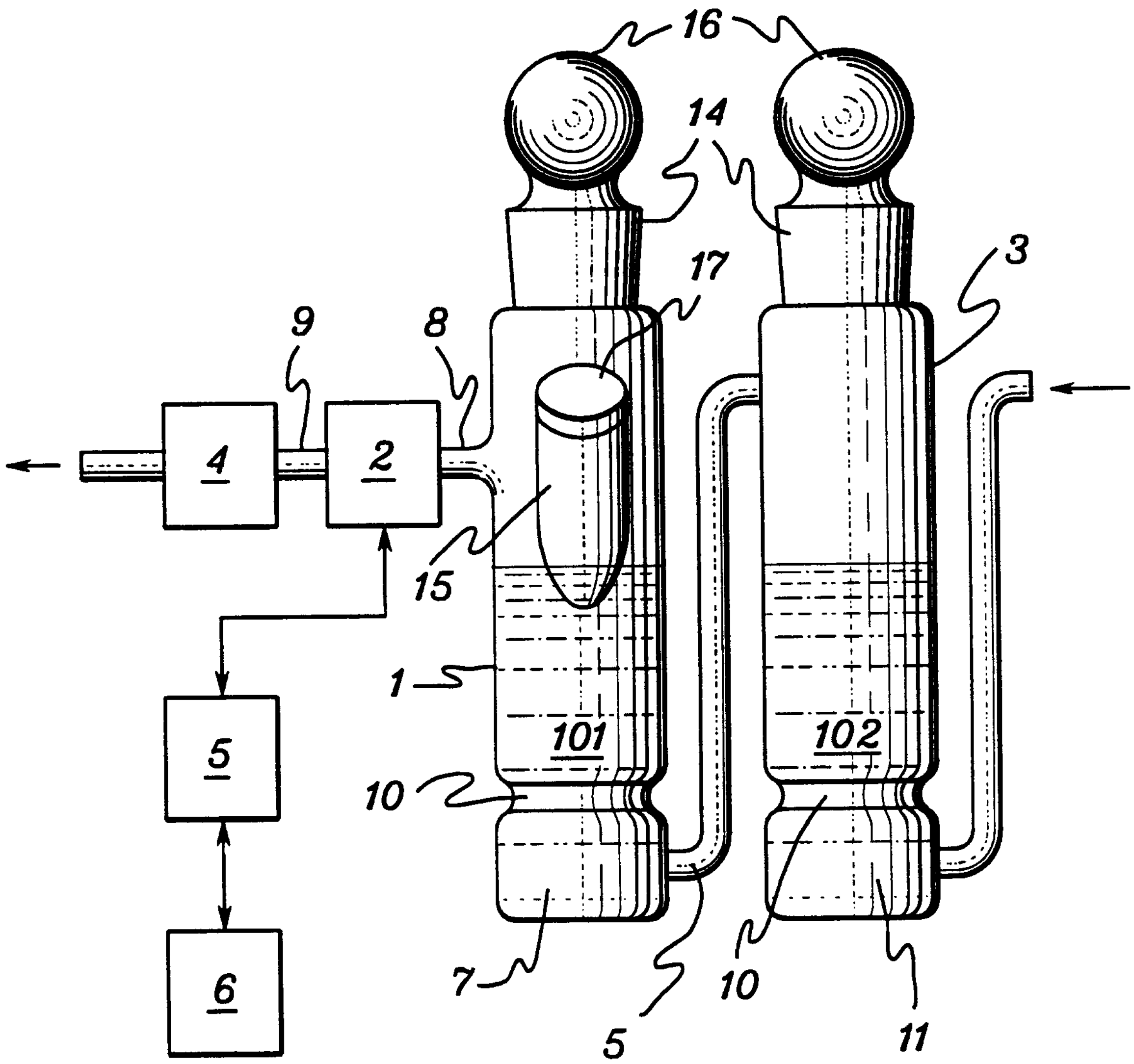


fig. 1

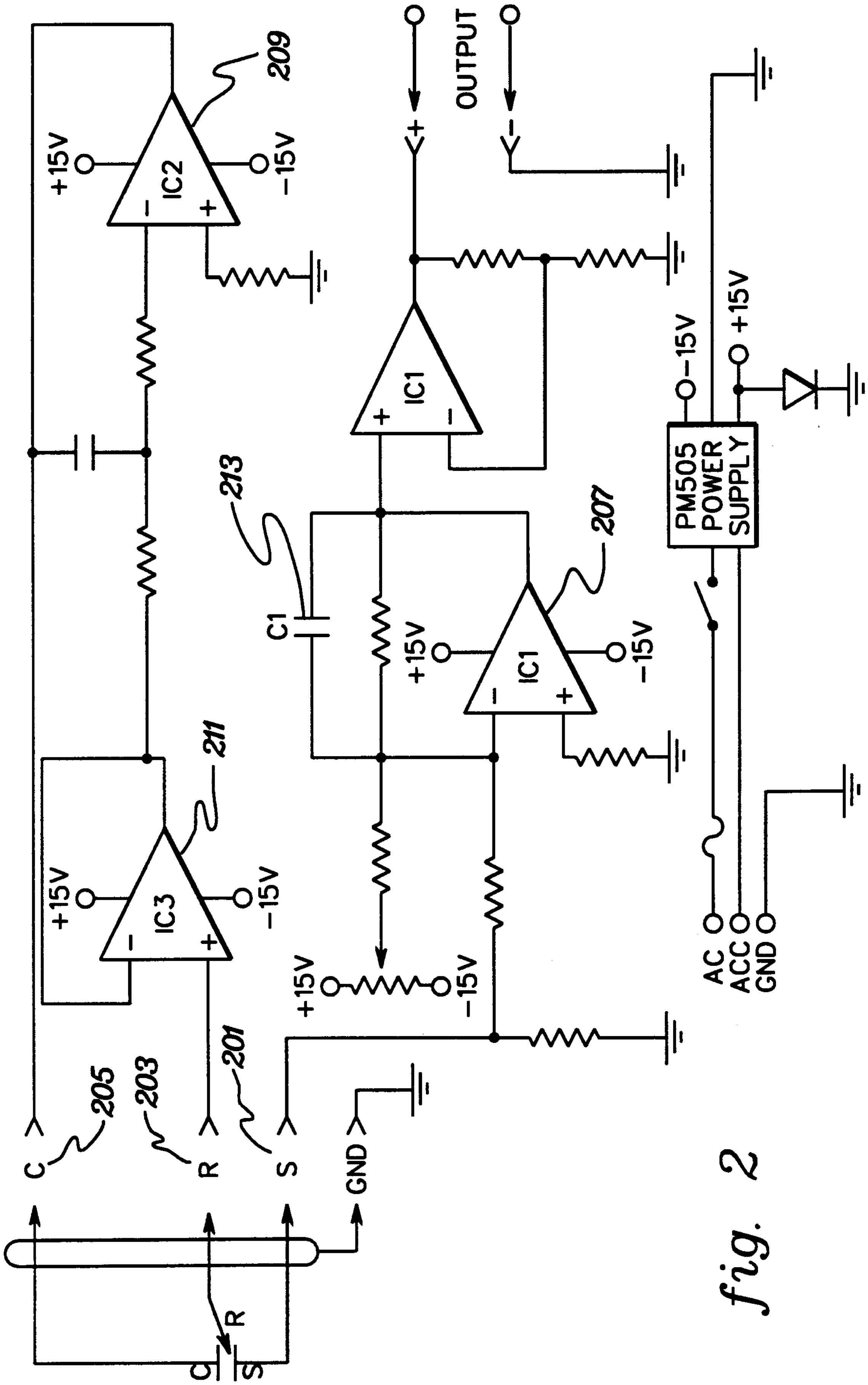
OUTPUT
+
-
IC2
209
+15V
-15V
IC1
213
C1
207
IC3
211
PM505
POWER
SUPPLY
AC
ACC
GND
C
R
S
205
203
201

fig. 2

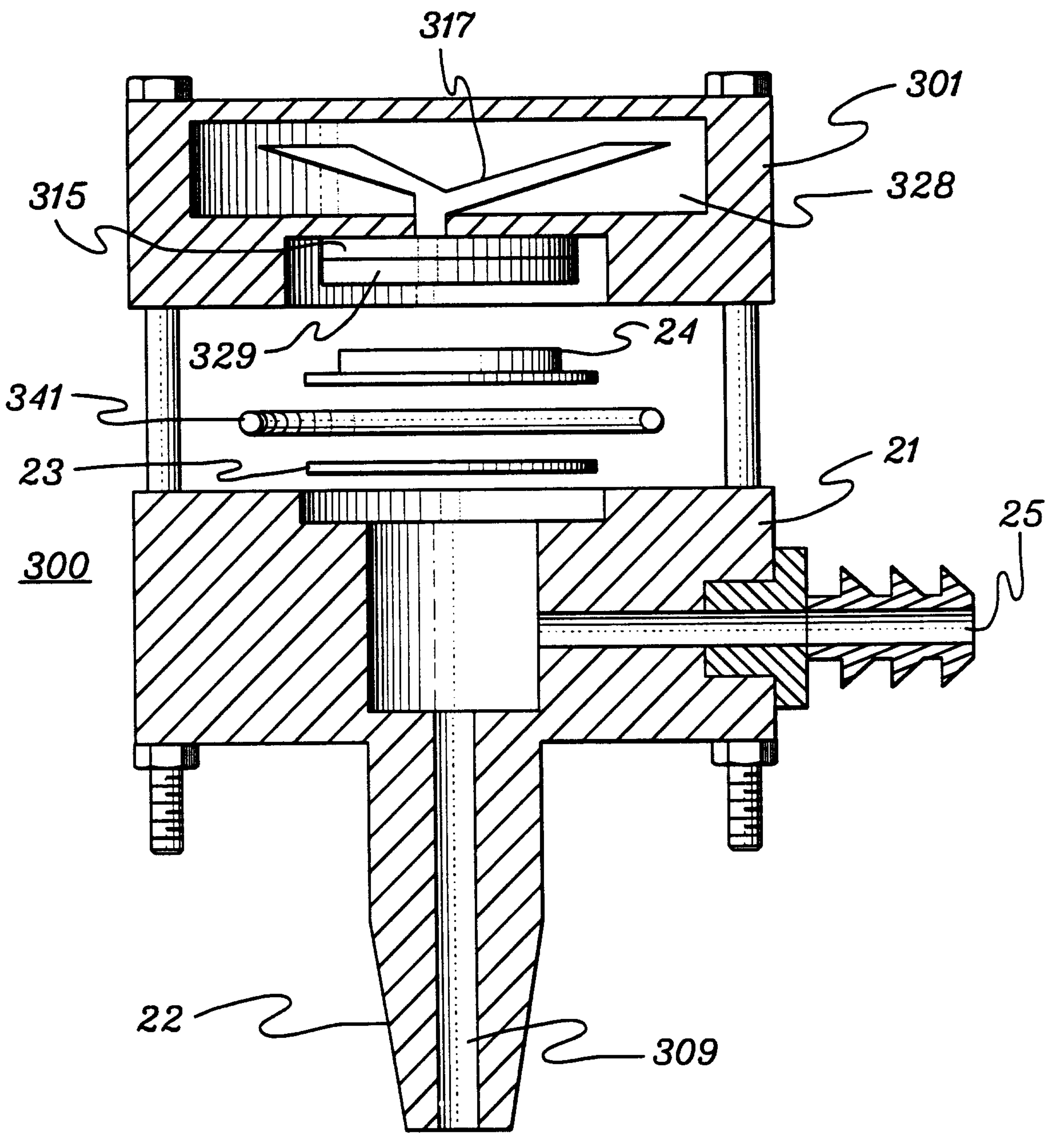


fig. 3

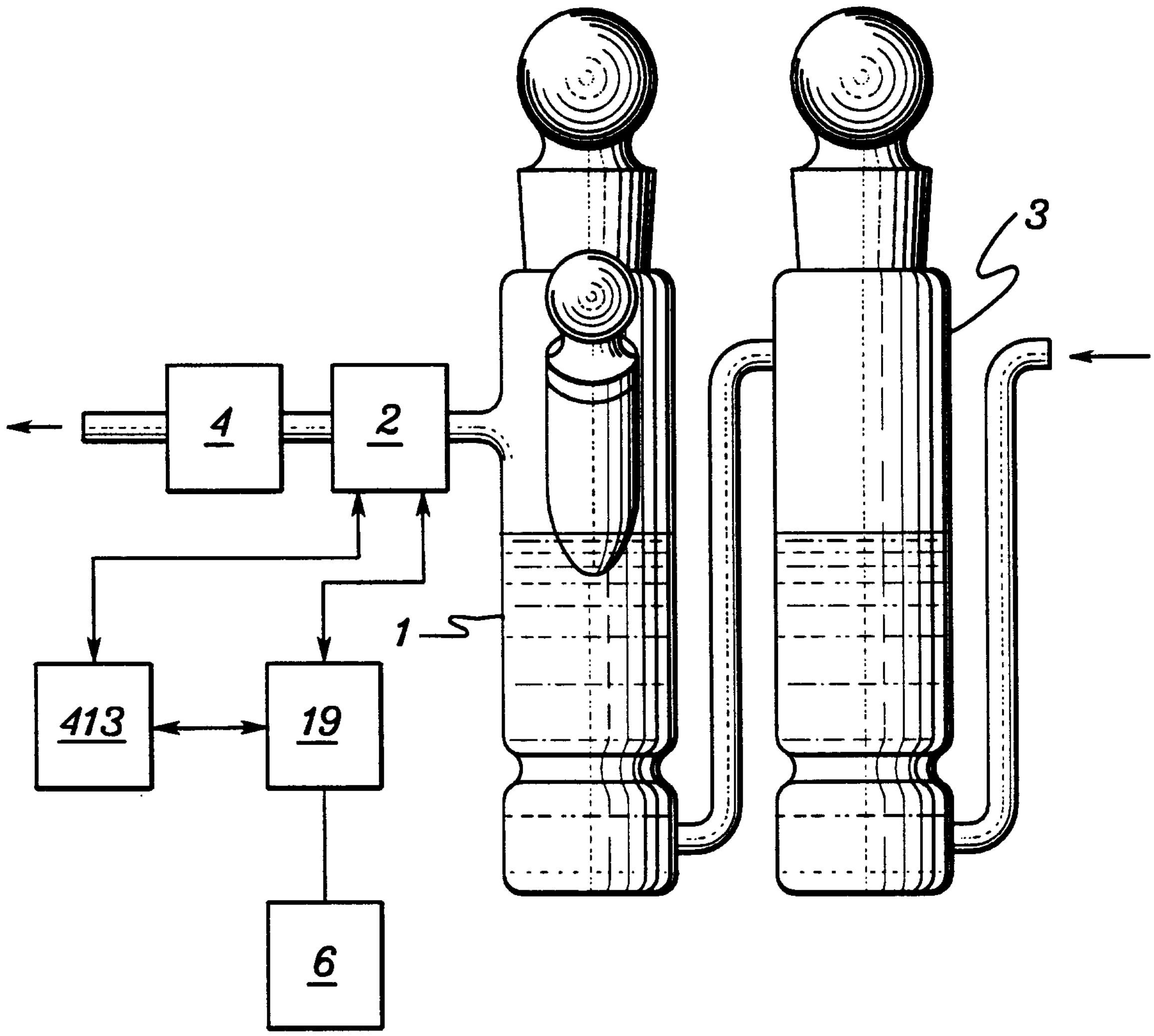


fig. 4

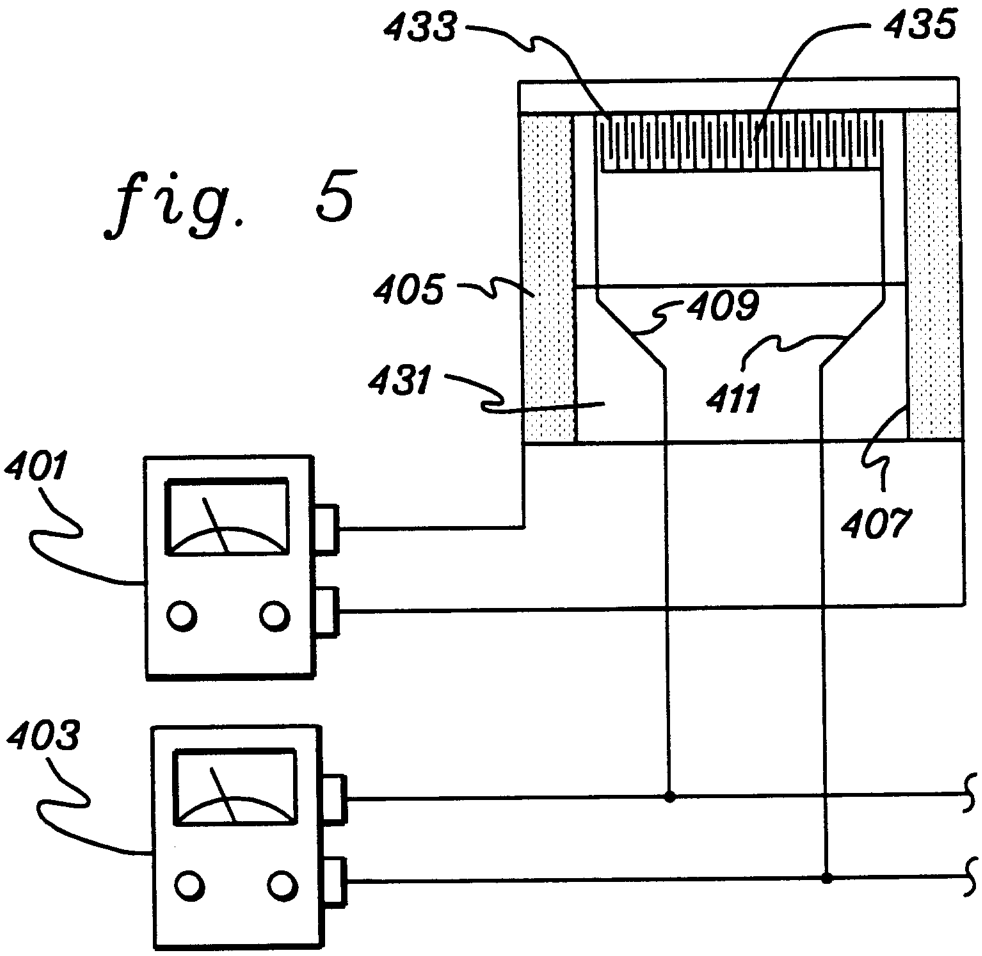


*fig. 5*

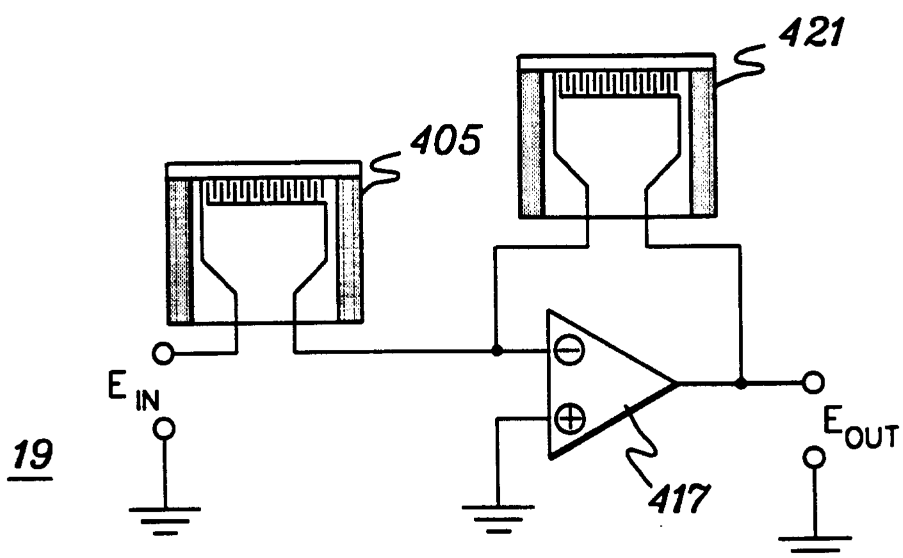


$$E_{OUT} = -E_{IN} \ (R_f / R_V)$$

*fig. 7*

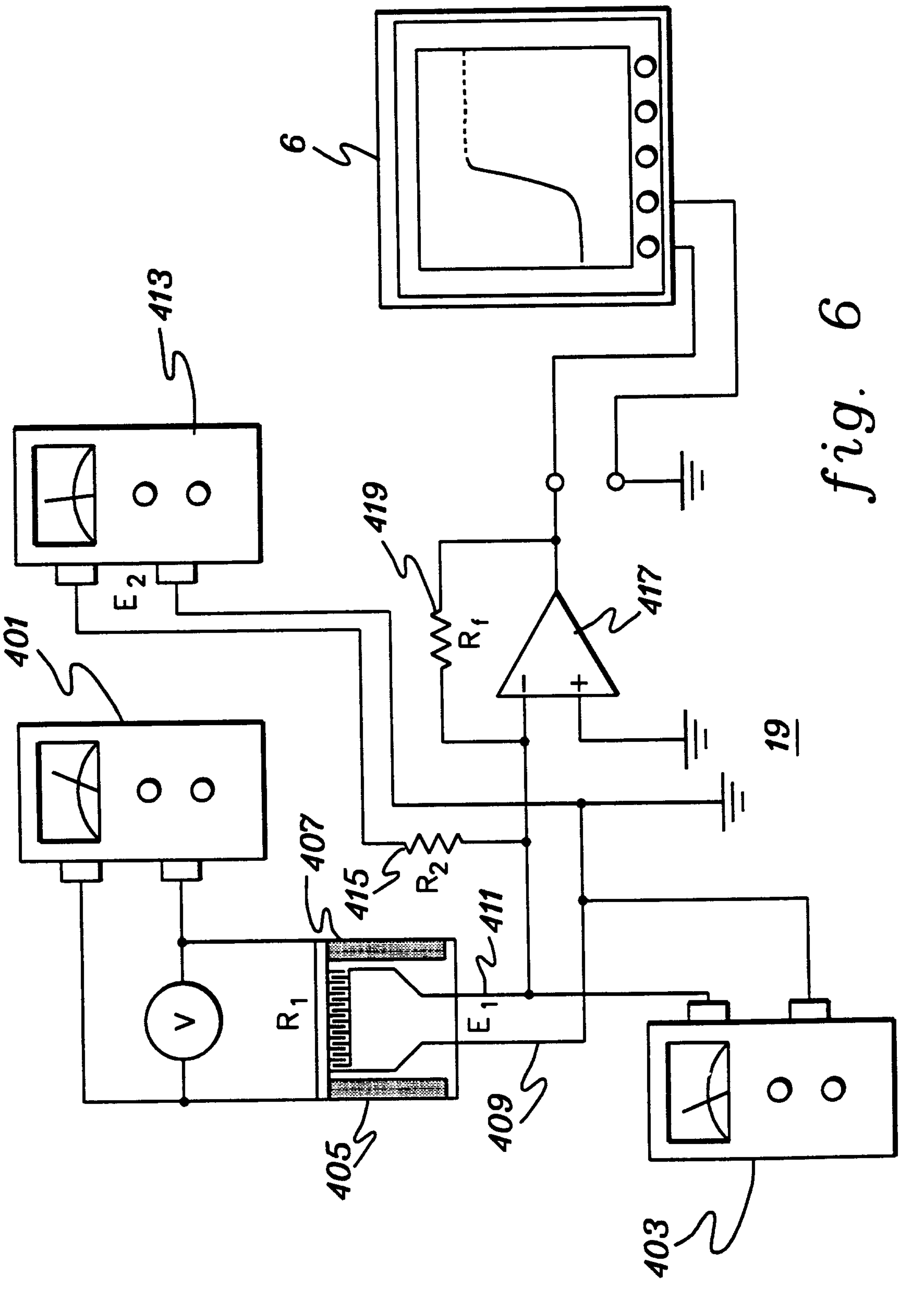


fig. 6

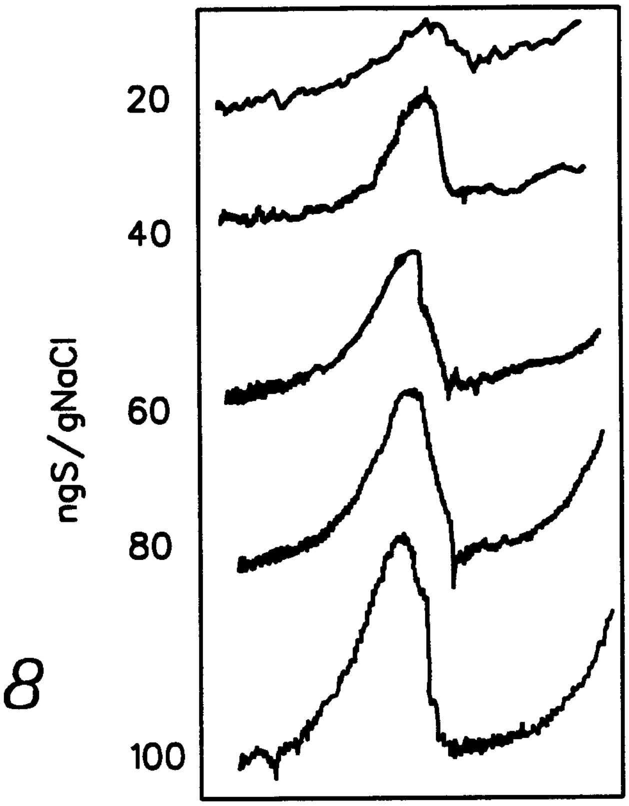
ngS/gNaCl
20
40
60
80
100

*fig. 8*

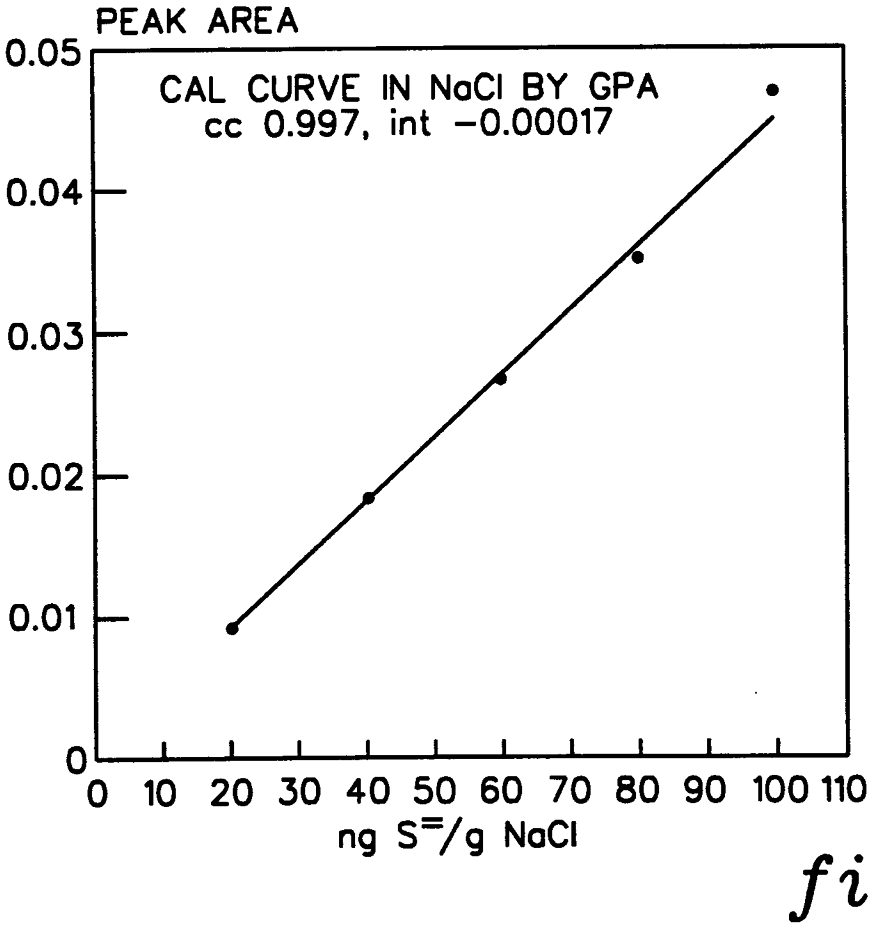
PEAK AREA
CAL CURVE IN NaCl BY GPA
cc 0.997, int −0.00017
0.05
0.04
0.03
0.02
0.01
0
0 10 20 30 40 50 60 70 80 90 100 110
ng S=/g NaCl

*fig. 9*

European Patent Office

# EUROPEAN SEARCH REPORT

Application Number

EP 93112075.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | WO - A1 -92/17 483 (EASTMAN KODAK COMPANY) * Totality * -- | 1-5 | G 01 N 27/12<br>G 01 N 31/00 |
| Y | US - A - 4 432 224 (TYPPO) * Column 4, lines 41-53; claims * -- | 1-5 | |
| D,Y | US - A - 5 034 246 (MANCE et al.) * Column 2, line 40 - column 4, line 59; examples; claims * -- | 1-5 | |
| A | EP - A - 0 252 627 (ENGLISH ELECTRIC VALVE COMPANY LIMITED) * Totality * | 1-7 | |
| D | & US-A-4 822 465 -- | | |
| A | EP - A - 0 952 277 (CITY TECHNOLOGY LIMITED) * Examples; claims * | 1-5 | TECHNICAL FIELDS SEARCHED (Int. Cl.5)<br>G 01 N<br>C 07 F<br>B 05 D |
| D | & US-A-4 633 704 ---- | | |

The present search report has been drawn up for all claims

| Place of search | Date of completion of the search | Examiner |
|---|---|---|
| VIENNA | 25-11-1993 | TENGLER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document